# EUROPEAN PATENT APPLICATION

(11) **EP 1 374 697 A1**
(43) Date of publication of application: **02.01.2004**
(21) Application number: 03253840.7
(22) Date of filing: 18.06.2003
(51) Int. Cl.: A23K 1/18, C12N 13/00, A61K 35/66, A23K 1/00, C12N 1/16

(54) **Feed additives for reducing odor of animal waste products**

(30) Priority: 18.06.2002 US 175014
(71) Applicant: Ultra Biotech Limited, Douglas IM1 1SA, Isle of Man (GB)
(72) Inventor: Cheung, Ling Yuk, New Territories, Hong Kong (CN)
(74) Representative: Kyle, Diana

(57) **Abstract**

The invention relates to biological compositions comprising yeast cells that can be added to animal feed which upon ingestion by an animal can reduce the odor of manure produced by the animal. The invention also relates to methods for manufacturing the biological compositions, and methods of using the biological compositions as animal feed additives.

## Description

### 1. FIELD OF THE INVENTION

The invention relates to biological compositions comprising yeast cells that can be added to animal feed which upon ingestion by an animal can reduce the odor of manure produced by the animal. The invention also relates to methods for manufacturing the biological compositions, and methods of using the biological compositions as animal feed additives.

### 2. BACKGROUND OF THE INVENTION

Animal agriculture in the United States produces almost $100 billion per year in farm revenue contributing to the vitality of rural communities and insuring the sustainability of America"s food supply (GAO, 1999). The U.S. has developed a very efficient, sophisticated system for production of meat, milk, poultry, and egg products involving concentrated animal feeding operations (CAFOs). Concentrated animal feeding operations (CAFOs), including swine and poultry operations, dairies and cattle feedlots and the associated animal waste management systems may produce emissions of odor, odorants, and odorous gases, such as ammonia, H₂S. For instance, in the United States in 1999, a monthly average of 10 million cattle were being finished for slaughter. During a normal 150 day finishing period, each animal excretes about 900 kg (2,000 lbs) of collectible manure, or about 1,800 kg/hd (4,000 lbs/hd) of manure per head of feedlot capacity per year. Cattle feedlots in the U.S. produce an estimated 18 million metric tons/yr (20 million tons/yr) of collectable manure containing at least 360,000 metric tons/yr (400,000 tons/yr) of total nitrogen and 135,000 metric tons/yr (150,000 tons/yr) of total phosphorus (P).

Historically, air quality protection has only received secondary consideration. Principal sources of odor emissions may include production facilities - open lot and confinement buildings; manure/wastewater storage and/or treatment systems - ponds, pits, lagoons, stockpiles, composting operations; and land application systems for solid or liquid manure, treated effluent, or open lot runoff.

Many technologies for control of odor and odorants from CAFOs have been developed over the last 3 or 4 decades. However, most of these technologies have not been evaluated properly or systematically. These measures generally fall under four broad approaches: (1) ration manipulation, (2) improved manure collection and treatment, (3) capture and treatment of odorous gases, and (4) enhanced dispersion.

Zhu et al. (1999) confirmed through an extensive literature review that most odorous compounds in swine manure are produced from processes involved in protein decomposition; and thus, reducing the protein content in the manure should help reduce swine manure odor. The addition of high dietary concentrations of copper to weaning and growing pigs has been shown to alter microflora patterns in the feces Goihl (2000), giving rise to the theory that subsequent odor of manure may be altered. The reduction of substrates for anaerobic activity is another approach to reducing odor emissions (Baidoo, 2000), and includes various feeding strategies such as: reduced nitrogen intake, phase feeding, repartitioning agents, improved animal genetics, and various feed additives. Some of the feed additives include: sugar beet pulp, soybean hulls, Jerusalem artichoke, zeolite, and yucca extracts.

A second approach involves manure treatment methods that include odor control measures, such as maintaining aerobic conditions during storage, aerobic treatment (aerated lagoons or composting), anaerobic digestion or biochemical treatment.

The capture-and-treat approach includes the use of covered storage pits or lagoons; soil incorporation of applied liquid or solid manure; and dry scrubbers for building exhaust gases, including soil absorption beds, bio-filter fields, or packed beds. Soils and organic materials such as peat or wood chips, have been used as they readily absorb odorous gases and provide for aerobic decomposition of captured odorants.

Odor control is of increasing concern. In the immediate future, application of those technologies available will be required to a greater extent (Miner, 1995). However, many of the technologies require expensive retrofits and precious land. It is also unclear whether some of the methods are environmentally sustainable systems from a nutrient management perspective.

The present invention provides a biological feed additive based on non-recombinant yeasts, which tackle the problem at the source in the animals' gastrointestinal tract. The present invention can reduce the problem of odor associated with processing, storing and using animal manure as a fertilizer.

Citation of documents herein is not intended as an admission that any of the documents cited herein is pertinent prior art, or an admission that the cited documents are considered material to the patentability of the claims of the present application. All statements as to the date or representations as to the contents of these documents are based on the information available to the applicant and does not constitute any admission as to the correctness of the dates or contents of these documents.

### 3. SUMMARY OF THE INVENTION

The present invention relates to biological compositions that can reduce the production of odorous waste products by animals. The biological compositions can be added to animal feed which are ingested by an animal and which reduce the odor of waste products prior to the shedding of the waste products by the animal.

In one embodiment, the present invention provides biological compositions comprising a plurality of live yeast cells which are capable of reducing the odor of the contents of the gastrointestinal tract of animals. The use of the biological compositions of the invention can result in a reduction of the environmental impact of animal wastes, as well as the processing and storing of manure.

In another embodiment, the invention provides methods of making the biological composition. In particular, the methods of the invention comprise culturing yeast cells in the presence of a series of electromagnetic fields of defined frequencies and field strengths, such that the yeast cells becomes metabolically active and potent at interacting with odorous compounds in the animal wastes. Up to six different components of yeast cells can be used to form the biological compositions. The yeast cells can also be subjected to a conditioning step to improve its performance. The conditioning step comprises culturing the yeast cells in a culture medium comprising animal gastric juice, wild hawthorn juice, and wild jujube juice. Methods for manufacturing the biological compositions comprising culturing the yeast cells under activation conditions, mixing various yeast cell cultures of the present invention, followed by drying the yeast cells and packing the final product, are encompassed. In preferred embodiments, the starting yeast cells are commercially available and/or accessible to the public, such as but not limited to *Saccharomyces cerevisiae.*

The biological compositions of the invention can be fed directly to animals or used as an additive to be incorporated into regular animal feed. Animal feed compositions comprising activated yeast cells of the invention and ingredients such as zeolite powder are encompassed by the invention.

### 4. BRIEF DESCRIPTION OF FIGURES

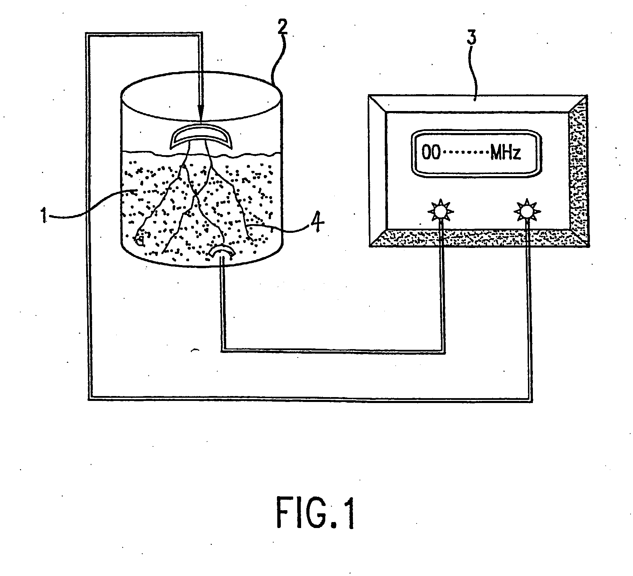

Fig. 1 Activation of yeast cells. 1 yeast cell culture; 2 container; 3 electromagnetic field source; 4 electrode..

### 5. DETAILED DESCRIPTION OF THE INVENTION

The present invention relates to biological compositions that can reduce the production of malodorous waste products by animals. The present invention provides methods for manufacturing the biological compositions as well as methods for using the biological compositions as animal feed additives.

The biological compositions of the invention comprise yeasts. Unlike the traditional use of yeasts as a component of the feed, the yeast cells of the invention are not a primary source of nutrients for the animals. The yeast cells of the invention can reduce the odor of wastes produced by animals. The use of the biological compositions of the invention can lower the overall cost of maintaining a commercial animal operations where manure is stored and processed to be used as a fertilizer.

While the following terms are believed to have well-defined meanings in the art, the following are set forth to define the terms as used herein, and facilitate explanation of the invention.

As used herein, the term "feed" broadly refers to any kind of material, liquid or solid, that is used for nourishing an animal, and for sustaining normal or accelerated growth of an animal including newborns and young developing animals. Preferably, the feed is pig feed.

The term "animal" as used herein refers to animals typically kept in farms, animal operations, CAFOs, zoos, and includes cattle, swine, sheep/goats, poultry (chicken and turkey).

As used herein, the phrase "reducing the odor of animal waste products or manure" refers to a process which results in a lower concentration of one or more malodorous compounds in animal waste products. Odorous compounds, such as but not limited to hydrogen sulfide, ammonia, indole, skatole (i.e, 3-methyl-1H-indole), p-cresol, and organic acids, are known to contribute to the malodorous quality of manure. The concentration of such malodorous compounds in manure or in a sample of air in contact with the manure can be determined by any method well known in the art, including but not limited to gas chromatography and mass spectroscopy, see e.g., Lacey 1998.

Odor is a perception of smell by an organism with olfactory organs. A reduction of the intensity of the odor associated with garbage can be determined subjectively. Various methods and techniques are known to measure the intensity of an odor. One subjective measurement of odor intensity is to measure the dilution necessary so that the odor is imperceptible or doubtful to a human or animal test panel. Alternatively, a recognition threshold may also be used which is a higher concentration at which the character of the odor is recognized. See, e.g., scentometer, dynamic olfactometers, suprathreshold referencing methods. Odor measurement methods that have been applied to animal waste management systems are described in Bulley and Phillips, 1980; Barth, et al., 1984; Watts, 1991; Sweeten, 1995; McFarland and Sweeten, 1995), which are incorporated by reference in their entireties. Any methods and techniques for objectively or subjectively determine the intensity of an odor can be used to monitor the performance of the compositions and methods of the invention.

In one embodiment, the present invention provides yeast cells that are capable of reducing the odor of animal waste products even before the wastes leave the animal's body. Without being bound by any theory, the inventor believes that the yeast cells of the invention are capable of reducing the odor of waste products by modifying or decomposing compounds present in an animal's intestine or in animal waste products, that are malodorous or that become malodorous. However, it is not necessary to demonstrate that such compounds have been modified. It is sufficient so long as the odor of the animal waste products is reduced as determined by any one of the methods described above, including subjectively by a panel of subjects, after the yeast cells of the invention have been used.

The present invention provides biological compositions that comprise at least one yeast cell component. Each yeast cell component comprises a population of live yeast cells which have been cultured under a specific set of conditions such that the yeast cells are capable of reducing the odor of a class of related malodorous compounds. In preferred embodiments, the biological compositions of the invention comprise up to six yeast cell components.

The biological compositions of the invention can be fed directly to an animal. In another embodiment, the biological compositions can be added to the feed. As known to those skilled in the relevant art, many methods and appliances may be used to mix the biological compositions of the invention with feed. In a particular embodiment, a mixture of culture broths of the yeasts of the present invention are added directly to the feed just prior to feeding the animal. Dried powders of the yeasts can also be added directly to the feed just prior to feeding the animal. In yet another embodiment of the present invention, the yeast cells are mixed with the raw constituents of the feed with which the yeast cells become physically incorporated. The biological compositions may be applied to and/or mixed with the feed by any mechanized means which may be automated.

The amount of biological composition used depends in part on the feeding regimen and the type of feed, and can be determined empirically. For example, the useful ratio of biological composition to animal feed ranges from 0.1% to 1% by dry weight, preferably, 0.3 to 0.8%, and most preferably at about 0.5%. Although not necessary, the biological compositions of the invention can also be used in conjunction or in rotation with other types of deodorants and nutrient supplements.

Described below in Section 5.1 and 5.2 are six yeast cell components of the invention and methods of their preparation. Section 5.3 describes the manufacture of the biological compositions of the invention which comprises at least one of the six yeast cell components.

### 5.1. PREPARATION OF THE YEAST CELL CULTURES

The present invention provides yeast cells that can reduce the production of malodorous waste products by an animal after having ingested the yeast cells. Up to six different yeast cell components can be combined to make the biological compositions.

According to the present invention, yeast cells that are capable of reducing the odor of organic materials are prepared by culturing the cells in the presence of an electromagnetic field in an appropriate culture medium. The frequency of the electromagnetic field for activating or enhancing this ability in yeasts can generally be found in the range of about 2140 to about 2462 MHz. After sufficient time is given for the yeast cells to grow, the yeast cells can be tested for their ability to reduce the odor of organic materials by methods well known in the art.

A yeast cell component of the biological composition is produced by culturing a plurality of yeast cells in an appropriate culture medium in the presence of an alternating electromagnetic field or multiple alternating electromagnetic fields in series over a period of time. The culturing process allows yeast spores to germinate, yeast cells to grow and divide, and can be performed as a batch process or a continuous process. As used herein, the terms "alternating electromagnetic field", "electromagnetic field" or "EM field" are synonymous. An electromagnetic field useful in the invention can be generated by various means well known in the art. A schematic illustration of exemplary setups are depicted respectively in Fig. 1. An electromagnetic field of a desired frequency and a desired field strength is generated by an electromagnetic wave source (3) which comprises one or more signal generators that are capable of generating electromagnetic waves, preferably sinusoidal waves, and preferably in the frequency range of 30 MHz - 3000 MHz. Such signal generators are well known in the art. Signal generators capable of generating signal with a narrower frequency range can also be used. If desirable, a signal amplifier can also be used to increase the output signal, and thus the strength of the EM field.

The electromagnetic field can be applied to the culture by a variety of means including placing the yeast cells in close proximity to a signal emitter connected to a source of electromagnetic waves. In one embodiment, the electromagnetic field is applied by signal emitters in the form of electrodes that are submerged in a culture of yeast cells (1). In a preferred embodiment, one of the electrodes is a metal plate which is placed on the bottom of a non-conducting container (2), and the other electrode comprises a plurality of wires or tubes so configured inside the container such that the energy of the electromagnetic field can be evenly distributed in the culture. The tips of the wires or tubes are placed within 3 to 30 cm from the bottom electrode plate (i.e, approximately 2 to 10% of the height of the container from the bottom). The number of electrode wires used depends on both the volume of the culture and the diameter of the wire. For example, for a culture having a volume of 10 liter or less, two ro three electrode wires having a diameter of between 0.5 to 2.0 mm can be used. For each 100 liter to 1000 liter of culture, the electrode wires or tubes can have a diameter of 6.0 to 15.0 mm. For a culture having a volume greater than 1000 liter, the electrode wires or tubes can have a diameter of between 20.0 to 25.0 mm.

In various embodiments, yeasts of the genera of *Saccharomyces, Candida, Crebrothecium, Geotrichum, Hansenula, Kloeckera, Lipomyces, Pichia, Rhodosporidium, Rhodotorula Torulopsis, Trichosporon,* and *Wickerhamia* can be used in the invention.

Non-limiting examples of yeast strains include *Saccharomyces cerevisiae* Hansen, ACCC2034, ACCC2035, ACCC2036, ACCC2037, ACCC2038, ACCC2039, ACCC2040, ACCC2041, ACCC2042, AS2.1, AS2.4, AS2.11, AS2.14, AS2.16, AS2.56, AS2.69, AS2.70, AS2.93, AS2.98, AS2.101, AS2.109, AS2.110, AS2.112, AS2.139, AS2.173, AS2.174, AS2.182, AS2.196, AS2.242, AS2.336, AS2.346, AS2.369, AS2.374, AS2.375, AS2.379, AS2.380, AS2.382, AS2.390, AS2.393, AS2.395, AS2.396, AS2.397, AS2.398, AS2.399, AS2.400, AS2.406, AS2.408, AS2.409, AS2.413, AS2.414, AS2.415, AS2.416, AS2.422, AS2.423, AS2.430, AS2.431, AS2.432, AS2.451, AS2.452, AS2.453, AS2.458, AS2.460, AS2.463, AS2.467, AS2.486, AS2.501, AS2.502, AS2.503, AS2.504, AS2.516, AS2.535, AS2.536, AS2.558, AS2.560, AS2.561, AS2.562, AS2.576, AS2.593, AS2.594, AS2.614, AS2.620, AS2.628, AS2.631, AS2.666, AS2.982, AS2.1190, AS2.1364, AS2.1396, IFFI 1001, IFFI 1002, IFFI 1005, IFFI 1006, IFFI 1008, IFFI 1009, IFFI 1010, IFFI 1012, IFFI 1021, IFFI 1027, IFFI 1037, IFFI 1042, IFFI 1043, IFFI 1045, IFFI 1048, IFFI 1049, IFFI 1050, IFFI 1052, IFFI 1059, IFFI 1060, IFFI 1063, IFFI 1202, IFFI 1203, IFFI 1206, IFFI 1209, IFFI 1210, IFFI 1211, IFFI 1212, IFFI 1213, IFFI 1215, IFFI 1220, IFFI 1221, IFFI 1224, IFFI 1247, IFFI 1248, IFFI 1251, IFFI 1270, IFFI 1277, IFFI 1287, IFFI 1289, IFFI 1290, IFFI 1291, IFFI 1292, IFFI 1293, IFFI 1297, IFFI 1300, IFFI 1301, IFFI 1302, IFFI 1307, IFFI 1308, IFFI 1309, IFFI 1310, IFFI 1311, IFFI 1331, IFFI 1335, IFFI 1336, IFFI 1337, IFFI 1338, IFFI 1339, IFFI 1340, IFFI 1345, IFFI 1348, IFFI 1396, IFFI 1397, IFFI 1399, IFFI 1411, IFFI 1413; *Saccharomyces cerevisiae* Hansen Var. ellipsoideus (Hansen) Dekker, ACCC2043, AS2.2, AS2.3, AS2.8, AS2.53, AS2.163, AS2.168, AS2.483, AS2.541, AS2.559, AS2.606, AS2.607, AS2.611, AS2.612; *Saccharomyces chevalieri* Guillennond, AS2.131, AS2.213; *Saccharomyces delbrueckii,* AS2.285; *Saccharomyces delbrueckii* Lindner var. mongolicus Lodder et van Rij, AS2.209, AS2.1157; *Saccharomyces exiguous* Hansen, AS2.349, AS2.1158; *Saccharomyces* fermentati (Saito) Lodder et van Rij, AS2.286, AS2.343; *Saccharomyces logos* van laer et Denamur ex Jorgensen, AS2.156, AS2.327, AS2.335; *Saccharomyces mellis* Lodder et Kreger Van Rij, AS2.195; *Saccharomyces microellipsoides* Osterwalder, AS2.699; Saccharomyces *oviformis* Osterwalder, AS2.100; *Saccharomyces rosei* (Guilliermond) Lodder et kreger van Rij, AS2.287; *Saccharomyces rouxii* Boutroux, AS2.178, AS2.180, AS2.370, AS2.371; *Saccharomyces sake* Yabe, ACCC2045; *Candida arborea,* AS2.566; *Candida Krusei* (Casteilani) Berkhout, AS2.1045; *Candida* lambica(Lindner et Genoud) van.Uden et Buckley, AS2.1182; *Candida lipolytica* (Harrison) Diddens et Lodder, AS2.1207, AS2.1216, AS2.1220, AS2.1379, AS2.1398, AS2.1399, AS2.1400; *Candida parapsilosis* (Ashford) Langeron et Talice, AS2.590; *Candida parapsilosis* (Ashford) et Talice Var. intermedia Van Rij et Verona, AS2.491; *Candida pulcherriman* (Lindner) Windisch, AS2.492; *Candida rugousa* (Anderson) Diddens et Loddeer, AS2.511, AS2.1367, AS2.1369, AS2.1372, AS2.1373, AS2.1377, AS2.1378, AS2.1384; *Candida tropicalis* (Castellani) Berkout, ACCC2004, ACCC2005, ACCC2006, AS2.164, AS2.402, AS2.564, AS2.565, AS2.567, AS2.568, AS2.617, AS2.1387; *Candida utilis* Henneberg Lodder et Kreger Van Rij, AS2.120, AS2.281, AS2.1180; *Crebrothecium ashbyii* (Guillermond) Routein, AS2.481, AS2.482, AS2.1197; *Geotrichum candidum* Link, ACCC2016, AS2.361, AS2.498, AS2.616, AS2.1035, AS2.1062, AS2.1080, AS2.1132, AS2.1175, AS2.1183; *Hansenula anomala* (Hansen) H et P sydow, ACCC2018, AS2.294, AS2.295, AS2.296, AS2.297, AS2.298, AS2.299, AS2.300, AS2.302, AS2.338, AS2.339, AS2.340, AS2.341, AS2.470, AS2.592, AS2.641, AS2.642, AS2.635, AS2.782, AS2.794; *Hansenula arabitolgens* Fang, AS2.887; *Hansenula jadinii* Wickerham, ACCC2019; *Hansenula saturnus* (Klocker) H et P sydow, ACCC2020; *Hansenula schneggii* (Weber) Dekker, AS2.304; *Hansenula subpelliculosa* Bedford, AS2.738, AS2.740, AS2.760, AS2.761, AS2.770, AS2.783, AS2.790, AS2.798, AS2.866; *Kloeckera apiculata* (Reess emend. Klocker) Janke, ACCC2021, ACCC2022, ACCC2023, AS2.197, AS2.496, AS2.711, AS2.714; *Lipomyces starkeyi* Lodder et van Rij, ACCC2024, AS2.1390; *Pichia* *farinosa* (Lindner) Hansen, ACCC2025, ACCC2026, AS2.86, AS2.87, AS2.705, AS2.803; *Pichia membranaefaciens* Hansen, ACCC2027, AS2.89, AS2.661, AS2.1039; *Rhodosporidium toruloides* Banno, ACCC2028; *Rhodotorula glutinis* (Fresenius) Harrison, ACCC2029, AS2.280, ACCC2030, AS2.102, AS2.107, AS2.278, AS2.499, AS2.694, AS2.703, AS2.704, AS2.1146; *Rhodotorula minuta* (Saito) Harrison, AS2.277; *Rhodotorula rubar* (Demme) Lodder, ACCC2031, AS2.21, AS2.22, AS2.103, AS2.105, AS2.108, AS2.140, AS2.166, AS2.167, AS2.272, AS2.279, AS2.282; *Saccharomyces carlsbergensis* Hansen, AS2.4, AS2.113, ACCC2032, ACCC2033, AS2.312, AS2.116, AS2.118, AS2.121, AS2.132, AS2.162, AS2.189, AS2.200, AS2.216, AS2.265, AS2.377, AS2.417, AS2.420, AS2.440, AS2.441, AS2.443, AS2.444, AS2.459, AS2.595, AS2.605, AS2.638, AS2.742, AS2.745, AS2.748, AS2.1042; *Saccharomyces uvarum Beijer,* IFFI 1023, IFFI 1032, IFFI 1036, IFFI 1044, IFFI 1072, IFFI 1205, IFFI 1207; *Saccharomyces willianus* Saccardo, AS2.5, AS2.7, AS2.119, AS2.152, AS2.293, AS2.381, AS2.392, AS2.434, AS2.614, AS2.1189; *Saccharomyces sp.,* AS2.311; *Saccharomyces ludwigii* Hansen, ACCC2044, AS2.243, AS2.508; *Saccharomyces sinenses* Yue, AS2.1395; *Schizosaccharomyces octosporus* Beijerinck, ACCC 2046, AS2.1148; *Schizosaccharomyces pombe* Linder, ACCC2047, ACCC2048, AS2.248, AS2.249, AS2.255, AS2.257, AS2.259, AS2.260, AS2.274, AS2.994, AS2.1043, AS2.1149, AS2.1178, IFFI 1056; *Sporobolomyces roseus* Kluyver et van Niel, ACCC 2049, ACCC 2050, AS2.619, AS2.962, AS2.1036, ACCC2051, AS2.261, AS2.262; *Torulopsis candida* (Saito) Lodder, ACCC2052, AS2.270; Torulopsis famta (Harrison) Lodder et van Rij, ACCC2053, AS2.685; *Torulopsis globosa* (Olson et Hammer) Lodder et van Rij, ACCC2054, AS2.202; *Torulopsis inconspicua* Lodder et van Rij, AS2.75; *Trichosporon behrendii* Lodder et Kreger van Rij, ACCC2055, AS2.1193; *Trichosporon capitatum* Diddens et Lodder, ACCC2056, AS2.1385; *Trichosporon cutaneum(de* Beurm et al.)Ota, ACCC2057, AS2.25, AS2.570, AS2.571, AS2.1374; *Wickerhamia fluoresens* (Soneda) Soneda, ACCC2058, AS2.1388. Yeasts of the *Saccharomyces* genus are generally preferred. Among strains of *Saccharomyces cerevisiae, Saccharomyces cerevisiae* Hansen is a preferred strain.

Generally, yeast strains useful for the invention can be obtained from private or public laboratory cultures, or publically accessible culture deposits, such as the American Type Culture Collection, 10801 University Boulevard, Manassas, VA 20110-2209 and the China General Microbiological Culture Collection Center (CGMCC), China Committee for Culture Collection of Microorganisms, Institute of Microbiology, Chinese Academy of Sciences, Haidian, P.O. Box 2714, Beijing, 100080, China.

Although it is preferred, the preparation of the yeast cell components of the invention is not limited to starting with a pure strain of yeast. Each yeast cell component may be produced by culturing a mixture of yeast cells of different species or strains. The constituents of a yeast cell component can be determined by standard yeast identification techniques well known in the art.

In various embodiments of the invention, standard techniques for handling, transferring, and storing yeasts are used. Although it is not necessary, sterile conditions or clean environments are desirable when carrying out the manufacturing processes of the invention. Standard techniques for handling animal blood and immune cells, and for studying immune functions of an animal are also used. Details of such techniques are described in Advances in Laboratory Methods: General Haematology, 2000, Assendelft et al., (Ed.), Arnold, Edward (Publisher); Handbook of Vertebrate Immunology, 1998, Pastoret et al. (Ed.), Academic Press, and Current Protocols In Immunology, 1991, Coligan, et al. (Ed), John Wiley & Sons, Inc., which are both incorporated herein by reference in their entireties.

In one embodiment, a method for producing yeast cells that reduce the amount of odorous organic acids, e.g., formic acid, acetic acid, propanoic acid, butyric acid, and other volatile fatty acids, is provided. Yeast cells that can reduce the odor of organic acids can be prepared by culturing the cells in the presence of at least one alternating electromagnetic (EM) field with a frequency in the range of 2442 MHz to 2462 MHz. A single EM field or a series of EM fields can be applied, each having a different frequency within the stated range, or a different field strength within the stated range, or different frequency and field strength within the stated ranges. Although any practical number of EM fields can be used within a series, it is preferred that, the yeast culture be exposed to a total of 2, 3, 4, 5, 6, 7, 8, 9 or 10 different EM fields in a series. The EM field(s), which can be applied by any means known in the art, can each have a frequency of 2442, 2443, 2444, 2445, 2446, 2447, 2448, 2449, 2450, 2451, 2452, 2453, 2454, 2455, 2456, 2457, 2458, 2459, 2460, 2461, or 2462 MHz.

The field strength of the EM field(s) is in the range of 25 to 300 mV/cm. In a preferred embodiment, the EM field(s) at the beginning of a series have a lower EM field strength than later EM field(s), such that the yeast cell culture are exposed to EM fields of progressively increasing field strength. Accordingly, the yeast cells can be cultured at the lower EM field strength (e.g., 120 to 150 mV/cm) for 20 to 80 hours and then cultured at the higher EM field strength (e.g., 250-300 mV/cm) for another 10 to 44 hours. The yeast culture can remain in the same container and use the same set of electromagnetic wave generator and emitters when switching from one EM field to another EM field.

The culture process can be initiated by inoculating 100ml of medium with 1ml of an inoculum of the selected yeast strain(s) at a cell density of about 10⁵ cells/ml. The starting culture is kept at 35 °C to 37°C for 24 to 48 hours prior to exposure to the EM field(s). The culturing process may preferably be conducted under conditions in which the concentration of dissolved oxygen is between 0.025 to 0.08 mol/m³, preferably 0.04 mol/m³. The oxygen level can be controlled by any conventional means known in the art, including but not limited to stirring and/or bubbling.

The culture is most preferably carried out in a liquid medium which contains animal serum and sources of nutrients assimilable by the yeast cells. Table 1 provides an exemplary medium for culturing the first yeast cell component of the invention.

**Table 1**

| Medium Composition | Quantity |
|---|---|
| Animal waste (dried weight) | 20.0g |
| NaCl | 0.3g |
| MgSO₄•7H₂O | 0.2g |
| CaCO₃•5H₂O | 0.5g |
| CaSO₄•2H₂O | 0.2g |
| K₂HPO₄ | 0.5g |
| Acetic acid / valeric acid mixture (equal parts at ≥ 100 µg/ml) | 600ml |
| Autoclaved water | 400ml |

The animal waste is obtained from the upper portion of the intestine of a freshly slaughtered animal. For convenience, waste from swine is generally preferred. Among the inorganic salts which can be incorporated in the culture media are the customary salts capable of yielding sodium, calcium, phosphate, sulfate, carbonate, and like ions. Non-limiting examples of nutrient inorganic salts are (MH₄)₂HPO₄, CaCO₃, MgSO₄, NaCl, and CaSO₄.

It should be noted that the composition of the media provided in Table 1 is not intended to be limiting. The process can be scaled up or down according to needs. Various modifications of the culture medium may be made by those skilled in the art, in view of practical and economic considerations, such as the scale of culture and local supply of media components.

Although the yeast cells will become activated even after a few hours of culturing in the presence of the EM field(s), the yeast cells can be cultured in the presence of the EM field(s) for an extended period of time (e.g., one week). At the end of the culturing process, the yeast cells which can be used as a yeast component of the invention may be recovered from the culture by various methods known in the art, and stored at a temperature below about 0°C to 4°C. The recovered yeast cells may also be dried and stored in powder form.

A non-limiting example of making a first yeast cell component of the invention with *Saccharomyces cerevisiae* strain AS2.558 is provided in Section 6 hereinbelow.

In another embodiment of the invention, a method for producing yeast cells that reduce the amount of ammonia and related ammonium containing compounds is provided. Yeast cells that remove ammonia and related ammonium containing compounds can be prepared by culturing the cells in the presence of at least one alternating electromagnetic (EM) field with a frequency in the range of 2225 MHz to 2245 MHz. A single EM field or a series of EM fields can be applied, each having a different frequency within the stated range, or a different field strength within the stated range, or different frequency and field strength within the stated ranges. Although any practical number of EM fields can be used within a series, it is preferred that, the yeast culture be exposed to a total of 2, 3, 4, 5, 6, 7, 8, 9 or 10 different EM fields in a series. The EM field(s), which can be applied by any means known in the art, can each have a frequency of 2225, 2226, 2227, 2228, 2229, 2230, 2231, 2232, 2233, 2234, 2235, 2236, 2237, 2238, 2239, 2240, 2241, 2242, 2243, 2244, or 2245 MHz.

The field strength of the EM field(s) is in the range of 25 to 300 mV/cm. In a preferred embodiment, the EM field(s) at the beginning of a series have a lower EM field strength than later EM field(s), such that the yeast cell culture are exposed to EM fields of progressively increasing field strength. Accordingly, the yeast cells can be cultured at the lower EM field strength (e.g., 150-200 mV/cm) for 36 to 88 hours and then cultured at the higher EM field strength (e.g., 240-270 mV./cm) for another 36 to 88 hours. The yeast culture can remain in the same container and use the same set of electromagnetic wave generator and emitters when switching from one EM field to another EM field.

The culture process can be initiated by inoculating 100ml of medium with 1ml of an inoculum of the selected yeast strain(s) at a cell density of about 10⁵ cells/ml. The starting culture is kept at 35°C to 37°C for 24 to 48 hours prior to exposure to the EM field(s). The culturing process may preferably be conducted under conditions in which the concentration of dissolved oxygen is between 0.025 to 0.08 mol/m³, preferably 0.04 mol/m³. The oxygen level can be controlled by any conventional means known in the art, including but not limited to stirring and/or bubbling.

The culture is most preferably carried out in a liquid medium which contains animal serum and sources of nutrients assimilable by the yeast cells. Table 2 provides an exemplary medium for culturing the second yeast cell component of the invention.

**Table 2**

| Medium Composition | Quantity |
|---|---|
| Animal waste (dried weight) | 20.0g |
| NaCl | 0.2g |
| MgSO₄•7H₂O | 0.2g |
| CaCO₃•5H₂O | 0.5g |
| CaSO₄•2H₂O | 0.2g |
| Peptone | 1.5g |
| K₂HPO₄ | 0.5g |
| Ammonia solution (at ≥ 100 µg/ml) | 600ml |
| Autoclaved water | 400ml |

The animal waste is obtained from an animal, such as a swine, as previously described. Among the inorganic salts which can be incorporated in the culture media are the customary salts capable of yielding sodium, calcium, phosphate, sulfate, carbonate, and like ions. Non-limiting examples of nutrient inorganic salts are (NH₄)₂HPO₄, CaCO₃, MgSO₄, NaCl, and CaSO₄.

It should be noted that the composition of the media provided in Table 2 is not intended to be limiting. The process can be scaled up or down according to needs. Various modifications of the culture medium may be made by those skilled in the art, in view of practical and economic considerations, such as the scale of culture and local supply of media components.

Although the yeast cells will become activated even after a few hours of culturing in the presence of the EM field(s), the yeast cells can be cultured in the presence of the EM field(s) for an extended period of time (e.g., two or more weeks). At the end of the culturing process, the yeast cells which constitute the first yeast cell component of the invention may be recovered from the culture by various methods known in the art, and stored at a temperature below about 0°C to 4°C. The recovered yeast cells may also be dried and stored in powder form.

A non-limiting example of making a second yeast cell component of the invention with *Saccharomyces cerevisiae* strain AS2.503 is provided in Section 6 hereinbelow.

In yet another embodiment, a method for producing yeast cells that reduce the amount of indole and other related compounds, such as skatol is provided. Yeast cells that reduce the amount of indole and other related compounds can be prepared by culturing the cells in the presence of at least one alternating electromagnetic (EM) field with a frequency in the range of 2320 MHz to 2340 MHz. A single EM field or a series of EM fields can be applied, each having a different frequency within the stated range, or a different field strength within the stated range, or different frequency and field strength within the stated ranges. Although any practical number of EM fields can be used within a series, it is preferred that, the yeast culture be exposed to a total of 2, 3, 4, 5, 6, 7, 8, 9 or 10 different EM fields in a series. The EM field(s), which can be applied by any means known in the art, can each have a frequency of 2320, 2321, 2322, 2323, 2324, 2325, 2326, 2327, 2328, 2329, 2330, 2331, 2332, 2333, 2334, 2335, 2336, 2337, 2338, 2339, or 2340 MHz.

The field strength of the EM field(s) is in the range of 25 to 300 mV/cm. In a preferred embodiment, the EM field(s) at the beginning of a series have a lower EM field strength than later EM field(s), such that the yeast cell culture are exposed to EM fields of progressively increasing field strength. Accordingly, the yeast cells can be cultured at the lower EM field strength (e.g., 125-200 mV/cm) for 36 to 100 hours and then cultured at the higher EM field strength (e.g., 220-240 mV/cm) for another 20 to 62 hours. The yeast culture can remain in the same container and use the same set of electromagnetic wave generator and emitters when switching from one EM field to another EM field.

The culture process can be initiated by inoculating 100ml of medium with 1ml of an inoculum of the selected yeast strain(s) at a cell density of about 10⁵ cells/ml. The starting culture is kept at 35°C to 37°C for 24 to 48 hours prior to exposure to the EM field(s). The culturing process may preferably be conducted under conditions in which the concentration of dissolved oxygen is between 0.025 to 0.08 mol/m³, preferably 0.04 mol/m³. The oxygen level can be controlled by any conventional means known in the art, including but not limited to stirring and/or bubbling.

The culture is most preferably carried out in a liquid medium which contains animal serum and sources of nutrients assimilable by the yeast cells. Table 3 provides an exemplary medium for culturing the third yeast cell component of the invention.

**Table 3**

| Medium Composition | Quantity |
|---|---|
| Animal waste (dried weight) | 20.0g |
| NaCl | 0.2g |
| MgSO₄•7H₂O | 0.2g |
| CaCO₃•5H₂O | 0.5g |
| CaSO₄•2H₂O | 0.2g |
| Peptone | 0.5g |
| K₂HPO₄ | 0.5g |
| Indole solution (≥ 100 µg/ml) | 600ml |
| Autoclaved water | 400ml |

The animal waste is obtained from the upper portion of the intestine of an animal, preferably a chicken. Among the inorganic salts which can be incorporated in the culture media are the customary salts capable of yielding sodium, calcium, phosphate, sulfate, carbonate, and like ions. Non-limiting examples of nutrient inorganic salts are (NH₄)₂HPO₄, CaCO₃, MgSO₄, NaCl, and CaSO₄.

It should be noted that the composition of the media provided in Table 3 is not intended to be limiting. The process can be scaled up or down according to needs. Various modifications of the culture medium may be made by those skilled in the art, in view of practical and economic considerations, such as the scale of culture and local supply of media components.

Although the yeast cells will become activated even after a few hours of culturing in the presence of the EM field(s), the yeast cells can be cultured in the presence of the EM field(s) for an extended period of time (e.g., two or more weeks). At the end of the culturing process, the yeast cells which constitute the first yeast cell component of the invention may be recovered from the culture by various methods known in the art, and stored at a temperature below about 0°C to 4°C. The recovered yeast cells may also be dried and stored in powder form.

A non-limiting example of making a third yeast cell component of the invention with *Saccharomyces cerevisiae* strain AS2.504 is provided in Section 6 hereinbelow.

In yet another embodiment, a method for producing yeast cells that reduce the amount of hydrogen sulfide and other related sulfur-containing or sulfhydryl (SH-) containing molecules is provided. Yeast cells that reduce the amount of hydrogen sulfide and other related sulfur-containing or sulfhydryl (SH-) containing molecules can be prepared by culturing the cells in the presence of at least one alternating electromagnetic (EM) field with a frequency in the range of 2230 MHz to 2250 MHz. A single EM field or a series of EM fields can be applied, each having a different frequency within the stated range, or a different field strength within the stated range, or different frequency and field strength within the stated ranges. Although any practical number of EM fields can be used within a series, it is preferred that, the yeast culture be exposed to a total of 2, 3, 4, 5, 6, 7, 8, 9 or 10 different EM fields in a series. The EM field(s), which can be applied by any means known in the art, can each have a frequency of 2230, 2231, 2232, 2233, 2234, 2235, 2236, 2237, 2238, 2239, 2240, 2241, 2242, 2243, 2244, 2245, 2246, 2247, 2248, 2249, or 2250 MHz.

The field strength of the EM fields) is in the range of 25 to 300 mV/cm. In a preferred embodiment, the EM field(s) at the beginning of a series have a lower EM field strength than later EM field(s), such that the yeast cell culture are exposed to EM fields of progressively increasing field strength. Accordingly, the yeast cells can be cultured at the lower EM field strength (e.g., 125-200 mV/cm) for 30 to 80 hours and then cultured at the higher EM field strength (e.g., 220-250 mV/cm) for another 10 to 54 hours. The yeast culture can remain in the same container and use the same set of electromagnetic wave generator and emitters when switching from one EM field to another EM field.

The culture process can be initiated by inoculating 100ml of medium with 1ml of an inoculum of the selected yeast strain(s) at a cell density of about 10⁵ cells/ml. The starting culture is kept at 35°C to 37°C for 24 to 48 hours prior to exposure to the EM field(s). The culturing process may preferably be conducted under conditions in which the concentration of dissolved oxygen is between 0.025 to 0.08 mol/m³, preferably 0.04 mol/m³. The oxygen level can be controlled by any conventional means known in the art, including but not limited to stirring and/or bubbling.

The culture is most preferably carried out in a liquid medium which contains animal serum and sources of nutrients assimilable by the yeast cells. Table 4 provides an exemplary medium for culturing the fourth yeast cell component of the invention.

**Table 4**

| Medium Composition | Quantity |
|---|---|
| Animal waste (dried weight) | 20.0g |
| NaCl | 0.2g |
| MgSO₄•7H₂O | 0.2g |
| CaCO₃•5H₂O | 0.5g |
| CaSO₄•2H₂O | 0.2g |
| Peptone | 1.5g or 0.3g, if ammonium sulfate is used |
| K₂HPO₄ | 0.5g |
| Hydrogen sulfide solution (≥ 100 µg/ml) | 600ml |
| Autoclaved water | 400ml |

The animal waste is obtained from the intestine of an animal, such as a swine, as previously described. Among the inorganic salts which can be incorporated in the culture media are the customary salts capable of yielding sodium, calcium, phosphate, sulfate, carbonate, and like ions. Non-limiting examples of nutrient inorganic salts are (NH₄)₂HPO₄, CaCO₃, MgSO₄, NaCl, and CaSO₄.

It should be noted that the composition of the media provided in Table 4 is not intended to be limiting. The process can be scaled up or down according to needs. Various modifications of the culture medium may be made by those skilled in the art, in view of practical and economic considerations, such as the scale of culture and local supply of media components.

Although the yeast cells will become activated even after a few hours of culturing in the presence of the EM field(s), the yeast cells can be cultured in the presence of the EM field(s) for an extended period of time (e.g., two or more weeks). At the end of the culturing process, the yeast cells which constitute the first yeast cell component of the invention may be recovered from the culture by various methods known in the art, and stored at a temperature below about 0°C to 4°C. The recovered yeast cells may also be dried and stored in powder form.

A non-limiting example of making a fourth yeast cell component of the invention with *Saccharomyces cerevisiae* strain AS2.443 is provided in Section 6 hereinbelow.

In yet another embodiment, a method for producing yeast cells that remove or degrade aliphatic substituted amine, such as methylamine, dimethylamine, or trimethylamine thereby reducing the odor caused by such compounds, is provided. Yeast cells that remove or degrade such amines can be prepared by culturing the cells in the presence of at least one alternating electromagnetic (EM) field with a frequency in the range of 2180 MHz to 2200 MHz. A single EM field or a series of EM fields can be applied, each having a different frequency within the stated range, or a different field strength within the stated range, or different frequency and field strength within the stated ranges. Although any practical number of EM fields can be used within a series, it is preferred that, the yeast culture be exposed to a total of 2, 3, 4, 5, 6, 7, 8, 9 or 10 different EM fields in a series. The EM field(s), which can be applied by any means known in the art, can each have a frequency of 2180, 2181, 2182, 2183, 2184, 2185, 2186, 2187, 2188, 2189, 2190, 2191, 2192, 2193, 2194, 2195, 2196, 2197, 2198, or 2200 MHz.

The field strength of the EM fields) is in the range of 25 to 300 mV/cm. In a preferred embodiment, the EM fields) at the beginning of a series have a lower EM field strength than later EM field(s), such that the yeast cell culture are exposed to EM fields of progressively increasing field strength. Accordingly, the yeast cells can be cultured at the lower EM field strength (e.g., 150-180 mV/cm) for 50 to 122 hours and then cultured at the higher EM field strength (e.g., 220-250 mV/cm) for another 15 to 54 hours. The yeast culture can remain in the same container and use the same set of electromagnetic wave generator and emitters when switching from one EM field to another EM field.

The culture process can be initiated by inoculating 100ml of medium with 1ml of an inoculum of the selected yeast strain(s) at a cell density of about 10⁵ cells/ml. The starting culture is kept at 35°C to 37°C for 24 to 48 hours prior to exposure to the EM field(s). The culturing process may preferably be conducted under conditions in which the concentration of dissolved oxygen is between 0.025 to 0.08 mol/m³, preferably 0.04 mol/m³. The oxygen level can be controlled by any conventional means known in the art, including but not limited to stirring and/or bubbling.

The culture is most preferably carried out in a liquid medium which contains animal serum and sources of nutrients assimilable by the yeast cells. Table 5 provides an exemplary medium for culturing the fourth yeast cell component of the invention.

**Table 5**

| Medium Composition | Quantity |
|---|---|
| Animal waste (dried weight) | 20.0g |
| NaCl | 0.2g |
| MgSO₄•7H₂O | 0.2g |
| CaCO₃•5H₂O | 0.5g |
| CaSO₄•2H₂O | 0.2g |
| Peptone | 0.2g |
| K₂HPO₄ | 0.5g |
| Dimethylamine/ethylenediamine mixture (equal parts at ≥ 100 µg/ml) | 600ml |
| Autoclaved water | 400ml |

The animal waste is obtained from the intestine of an animal, such as a swine, as previously described. Among the inorganic salts which can be incorporated in the culture media are the customary salts capable of yielding sodium, calcium, phosphate, sulfate, carbonate, and like ions. Non-limiting examples of nutrient inorganic salts are (NH₄)₂HPO₄, CaCO₃, MgSO₄, NaCl, and CaSO₄.

It should be noted that the composition of the media provided in Table 5 is not intended to be limiting. The process can be scaled up or down according to needs. Various modifications of the culture medium may be made by those skilled in the art, in view of practical and economic considerations, such as the scale of culture and local supply of media components.

Although the yeast cells will become activated even after a few hours of culturing in the presence of the EM field(s), the yeast cells can be cultured in the presence of the EM field(s) for an extended period of time (e.g., one or more weeks). At the end of the culturing process, the yeast cells which constitute the first yeast cell component of the invention may be recovered from the culture by various methods known in the art, and stored at a temperature below about 0°C to 4°C. The recovered yeast cells may also be dried and stored in powder form.

A non-limiting example of making a fifth yeast cell component of the invention with *Saccharomyces cerevisiae* strain AS2.420 is provided in Section 6 hereinbelow.

In yet another embodiment, a method for producing yeast cells that reduce the amount of p-cresol and related compounds in animal waste is provided. Yeast cells that reduce the amount of p-cresol and other related compounds can be prepared by culturing the cells in the presence of at least one alternating electromagnetic (EM) field with a frequency in the range of 2245 MHz to 2265 MHz. A single EM field or a series of EM fields can be applied, each having a different frequency within the stated range, or a different field strength within the stated range, or different frequency and field strength within the stated ranges. Although any practical number of EM fields can be used within a series, it is preferred that, the yeast culture be exposed to a total of 2, 3, 4, 5, 6, 7, 8, 9 or 10 different EM fields in a series. The EM field(s), which can be applied by any means known in the art, can each have a frequency of 2245, 2246, 2247, 2248, 2249, 2250, 2251, 2252, 2253, 2254, 2255, 2256, 2257, 2258, 2259, 2260, 2261, 2262, 2263, 2264, or 2265 MHz.

The field strength of the EM field(s) is in the range of 25 to 300 mV/cm. In a preferred embodiment, the EM field(s) at the beginning of a series have a lower EM field strength than later EM field(s), such that the yeast cell culture are exposed to EM fields of progressively increasing field strength. Accordingly, the yeast cells can be cultured at the lower EM field strength (e.g., 150-180 mV/cm) for 30 to 88 hours and then cultured at the higher EM field strength (e.g., 180-200 mV/cm) for another 30 to 88 hours. The yeast culture can remain in the same container and use the same set of electromagnetic wave generator and emitters when switching from one EM field to another EM field.

The culture process can be initiated by inoculating 100ml of medium with 1ml of an inoculum of the selected yeast strain(s) at a cell density of about 10⁵ cells/ml. The starting culture is kept at 35°C to 37°C for 24 to 48 hours prior to exposure to the EM field(s). The culturing process may preferably be conducted under conditions in which the concentration of dissolved oxygen is between 0.025 to 0.08 mol/m³, preferably 0.04 mol/m³. The oxygen level can be controlled by any conventional means known in the art, including but not limited to stirring and/or bubbling.

The culture is most preferably carried out in a liquid medium which contains animal serum and sources of nutrients assimilable by the yeast cells. Table 6 provides an exemplary medium for culturing the fourth yeast cell component of the invention.

**Table 6**

| Medium Composition | Quantity |
|---|---|
| Animal waste (dried weight) | 20.0g |
| NaCl | 0.2g |
| MgSO₄•7H₂O | 0.2g |
| CaCO₃•5H₂O | 0.5g |
| CaSO₄•2H₂O | 0.2g |
| Peptone | 1.5g |
| K₂HPO₄ | 0.5g |
| P-cresol solution (≥ 100 ug/ml) | 600ml |
| Autoclaved water | 400ml |

The animal waste is obtained from the intestine of an animal, such as a swine, as previously described. Among the inorganic salts which can be incorporated in the culture media are the customary salts capable of yielding sodium, calcium, phosphate, sulfate, carbonate, and like ions. Non-limiting examples of nutrient inorganic salts are (NH₄)₂HPO₄, CaCO₃, MgSO₄, NaCl, and CaSO₄.

It should be noted that the composition of the media provided in Table 6 is not intended to be limiting. The process can be scaled up or down according to needs. Various modifications of the culture medium may be made by those skilled in the art, in view of practical and economic considerations, such as the scale of culture and local supply of media components.

Although the yeast cells will become activated even after a few hours of culturing in the presence of the EM field(s), the yeast cells can be cultured in the presence of the EM field(s) for an extended period of time (e.g., one or more weeks). At the end of the culturing process, the yeast cells which constitute the first yeast cell component of the invention may be recovered from the culture by various methods known in the art, and stored at a temperature below about 0°C to 4°C. The recovered yeast cells may also be dried and stored in powder form.

A non-limiting example of making a sixth yeast cell component of the invention with *Saccharomyces carlsbergensis* strain AS2.4 is provided in Section 6 hereinbelow.

### 5.2. CONDITIONING OF THE YEAST CELLS

In another aspect of the invention, performance of the activated yeast cells can be optimized by culturing the activated yeast cells in the presence of materials taken from the gastrointestinal tract of the type of animal to which the biological composition will be fed. The inclusion of this conditioning process allows the activated yeast cells to adapt to and endure the acidic environment of the animal's stomach.

According to the invention, activated yeast cells prepared as described in Section 5.1 (for example, AS2.558, AS2.503, AS2.504, AS2.443, AS2.420 and AS2.4) can be further cultured in a medium with a composition as shown in Table 7.

**Table 7**

| (Per 1000 ml of culture medium) | |
|---|---|
| Medium Composition | Quantity |
| Porcine gastric juice | 100ml; stored at 4°C |
| Extract of swine waste | 200ml |
| Wild jujube juice | 300ml |
| Wild hawthorn juice | 320ml |
| (NH₄)₂HPO₄ | 0.25g |
| K₂HPO₄ | 0.2g |
| MgSO₄•7H₂O | 0.22g |
| NaCl | 0.5g |
| CaSO₄•2H₂O | 0.3g |
| CaCO₃•5H₂O | 3.0g |
| Yeast cultures from each of the six yeast cell components containing > 10⁸ cells/ml | 20 ml each; a total of 120 ml |

The process can be scaled up or down according to needs.

The gastric juice of the animal, for example, a pig, can be obtained from the liquid portion of the stomach content of a freshly slaughtered animal. The content of the stomach is filtered under sterile conditions to obtain a clear fluid which can be stored at 4°C before use.

The extract of swine waste is prepared by mixing 500 g of swine waste with 1000ml of water, allowing the sedimentation for 24 hours in room temperature and taking the supernatant of the mixture for use in the process.

The wild jujube juice is a filtered extract of wild jujube fruits prepared by mixing 5 ml of water per gram of crushed wild jujube. The wild hawthorn juice is a filtered extract of wild hawthorn fruits prepared by mixing 5 ml of water per gram of crushed wild hawthorn.

The mixture of yeast cells is cultured for about 36 hours in the presence of a series of electromagnetic fields. Each electromagnetic field has a frequency that, depending on the strains of yeast included, corresponds to one of the six ranges of frequencies described in Sections 5.1. If all six yeast components are present, a combination of the following six frequency bands can be used : 2442-2462 MHz; 2225-2245 MHz; 2320-2340 MHz; 2230-2250 MHz; 2180-2200 MHz; and 2245-2265 MHz. The EM fields can be applied sequentially or simultaneously. Generally, the yeast cells are subjected to an EM field strength in the range of about 312mV/cm in this process.

While the yeast cell culture is exposed to the EM field(s), the culture is incubated at temperatures that is maintained at about 35 °C to about 37°C.

### 5.3 MANUFACTURE OF THE BIOLOGICAL COMPOSITIONS

The present invention further provides a method for manufacturing a biological composition that comprises the yeast cells of the invention. Preferably, the biological compositions of the invention comprise yeast cells activated by the methods described in section 5.1 and which have been subject to conditioning by the method described in section 5.2. Most preferably, the biological compositions comprise all six yeast cell components.

To mass produce the biological compositions of the invention, the culture process is scaled up accordingly. To illustrate the scaled-up process, a method for producing 1000 kg of the biological composition is described as follows:

A stock culture of each of the six yeast cell components are added to a culture medium comprising 100 kg starch in about 230 liters of water. The yeast cells are then cultured at 35 ° to 37 °C in the presence of an EM field(s) of the respective frequencies and a field strength about 245mV/cm. The culture process is carried out for about 32 hours, or when the yeast cell number reaches about 2 x 10¹⁰/ml. At this point, the yeast cells must be stored at about 15 ° to 20°C, and if not used immediately, dried for storage within 24 hours. This process is repeated for each of the six yeast cell components. To make a biological composition comprising all six yeast cell components, 250 liters of culture media of each of the six yeast cell components (i.e, a total of 1500 liters) are mixed and combined with 600 kg of starch.

Since the yeast cells and the biological compositions are not necessarily used immediately, the prepared yeast cells and biological compositions can be dried in a two-stage drying process. During the first drying stage, the yeast cells are dried in a first dryer at a temperature not exceeding 65 ° C for a period of time not exceeding 10 minutes so that yeast cells quickly become dormant. The yeast cells are then sent to a second dryer and dried at a temperature not exceeding 70°C for a period of time not exceeding 30 minutes to further remove water. After the two stages, the water content should be lower than 5%. It is preferred that the temperatures and drying times be adhered to in both drying stages so that yeast cells do not lose their vitality and functions. The dried yeast cells are then cooled to room temperature. The dried yeast cells may also be screened in a separator so that particles of a preferred size are selected. The dried cells can then be sent to a bulk bag filler for packing.

### 6. EXAMPLE

The following example illustrates the manufacture of a biological composition that can be used as an animal feed additive.

A biological composition of the invention can comprise any one or more of the following six components of yeasts: *Saccharomyces cerevisiae* AS2.558, AS2.503, AS2.504, AS2.443, AS2.420 and AS2.4. The six yeast cell components are prepared and tested separately. Each of the six components have been shown to reduce the amount of malodorous compounds. To directly determine the activity of the activated yeast cells towards an malodorous compounds, animal wastes were mixed with various malodorous compounds and the mixture was incubated with activated yeast cells in an environment that approximates the gastrointestinal tract of an animal. After incubation, high performance liquid chromatography was used to measure the amounts of malodorous compounds. remaining in the test mixture. Known standards were also used to aid determining quantitatively and qualitatively the composition of the test mixture.

Accordingly to the invention, the odor caused by organic acids (such as formic acid, acetic acid, propanoic acid, butyric acid, and other volatile fatty acids) can be reduced by yeasts cultured in the presence of an EM field that is in the range of 2442 to 2462 MHz. To demonstrate this aspect of the invention, cells of *Saccharomyces cerevisiae* strain AS2.558 were cultured in a medium as described in Table 1 for 28 hours at 36°C. The culture started with 10⁹ cells per liter of medium. After the initial period of culture, the yeast cells were cultured in the presence of a series of eight EM fields in the order stated: 2442MHz at 146mv/cm for 22 hrs; 2448MHz at 146mv/cm for 22 hrs; 2452MHz at 146mv/cm for 10 hrs; 2460MHz at 146mv/cm for 10 hrs 2442MHz at 265mv/cm for 18 hrs; 2448MHz at 265mv/cm for 18 hrs; 2452MHz at 265mv/cm for 12 hrs; 2460MHz at 265mv/cm for 12 hrs. At the end of the culture period, the yeast cells were dried as described above.

To test the activated AS2.558 cells, a malodorous mixture was prepared as follow. 500 g of swine waste was collected and placed in a flask in which a partial vacuum is created. Two ml each of acetic acid, propanoic acid and valeric acid (each >95% pure) and 300 ml of water were added to and mixed with the animal waste under the partial vacuum. The partial vacuum is created by withdrawing about a volume of air which is about 20% of the volume of air above the surface of the extract in the flask. Nine such flasks were prepared. About 100 µl of activated AS2.558 yeast cells containing about 10⁹ cells were added to each of three flasks. To another three flasks were added the same number of non-activated AS2.558 yeast cells. No yeast cells were added to the remaining three flasks. The nine flasks were incubated for 37°C for 3 hours. HPLC was used to analyze the amount of organic acids in the mixture immediately after incubation. The amount of organic acid was reduced by more than 92.7% relative to the control containing no yeasts. There was no significant changes in the samples containing the non-activated yeasts or blank controls.

Accordingly to the invention, the odor caused by ammonia and related ammonium containing compounds can be reduced by yeasts cultured in the presence of an EM field that is in the range of 2225 to 2245 MHz. To demonstrate this aspect of the invention, cells of *Saccharomyces cerevisiae* strain AS2.503 were cultured in a medium as described in Table 1 for 28 hours at 36°C. The culture started with 10⁹ cells per liter of medium. After the initial period of culture, the yeast cells were cultured in the presence of a series of eight EM fields in the order stated: 2226 MHz at 187 mV/cm for 38 hrs; 2233 MHz at 187 mV/cm for 38 hrs; 2239 MHz at 187 mV/cm for 8 hrs; 2243 MHz at 187 mV/cm for 8 hrs; 2226MHz at 250mv/cm for 36 hrs; 2233MHz at 250mv/cm for 36 hrs; 2239MHz at 250mv/cm for 8 hrs; and 2243MHz at 250mv/cm for 8 hrs. At the end of the culture period, the yeast cells were dried as described above.

To test the activated AS2.503 cells, a malodorous mixture was prepared as follow. 500 g of chicken waste was collected and placed in a flask in which a partial vacuum is created. Five ml of ammonia solution (80% NH₃-N) and 300 ml of water were added to and mixed with the animal waste under the partial vacuum. The partial vacuum is created by withdrawing about a volume of air which is about 20% of the volume of air above the surface of the extract in the flask. Nine such flasks were prepared. About 100 µl of activated AS2.503 yeast cells containing about 10⁹ cells were added to each of three flasks. To another three flasks were added the same number of non-activated AS2.503 yeast cells. No yeast cells were added to the remaining three flasks. The nine flasks were incubated for 37°C for 3 hours in an enclosed atmosphere with oxygen.

HPLC was used to analyze the amount of ammonia in the mixture immediately after incubation. The amount of ammonia was reduced by more than 84.7% relative to the control containing no yeasts. There were no significant changes in the samples containing non-activated yeasts and the blank control.

Accordingly to the invention, the odor caused by indole and other related molecules can be reduced by yeasts cultured in the presence of an EM field that is in the range of 2320 to 2340 MHz. To demonstrate this aspect of the invention, cells of *Saccharomyces cerevisiae* strain AS2.504 were cultured in a medium as described in Table 1 for 28 hours at 36°C. The culture started with 10⁹ cells per liter of medium. After the initial period of culture, the yeast cells were cultured in the presence of a series of eight EM fields in the order stated: 2321MHz at 189mv/cm for 42 hrs; 2325MHz at 189mv/cm for 42 hrs; 2342MHz at 189mv/cm for 8 hrs; 2349MHz at 189mv/cm for 8 hrs; 2321MHz at 234mv/cm for 26 hrs; 2325MHz at 234mv/cm for 26 hrs; 2342MHz at 234mv/cm for 5 hrs; 2349MHz at 234mv/cm for 5 hrs. At the end of the culture period, the yeast cells were dried as described above.

To test the activated AS2.504 cells, a malodorous mixture was prepared as follow. 500 g of swine waste was collected and placed in a flask in which a partial vacuum is created. Five ml of liquid indole (85% pure) and 300 ml of water were added to and mixed with the animal waste under the partial vacuum. The partial vacuum is created by withdrawing about a volume of air which is about 20% of the volume of air above the surface of the extract in the flask. Nine such flasks were prepared. About 100 µl of activated AS2.504 yeast cells containing about 10⁹ cells were added to each of three flasks. To another three flasks were added the same number of non-activated AS2.504 yeast cells. No yeast cells were added to the remaining three flasks. The nine flasks were incubated for 37°C for 3 hours in an enclosed atmosphere with oxygen.

HPLC was used to analyze the amount of indole in the mixture immediately after incubation. The amount of indole was reduced by more than 79.6% relative to the control containing no yeasts. There were no significant changes in the samples containing non-activated yeasts and the blank control.

Accordingly to the invention, the odor caused by hydrogen sulfide and other related sulfur-containing or sulfhydryl (SH-) containing molecules can be reduced by yeasts cultured in the presence of an EM field that is in the range of 2230 to 2250 MHz. To demonstrate this aspect of the invention, cells of *Saccharomyces cerevisiae* strain AS2.443 were cultured in a medium as described in Table 1 for 28 hours at 36°C. The culture started with 10⁹ cells per liter of medium. After the initial period of culture, the yeast cells were cultured in the presence of a series of eight EM fields in the order stated: 2230MHz at 192mv/cm for 35 hrs; 2237MHz at 192mv/cm for 35 hrs; 2241MHz at 192mv/cm for 5 hrs; 2244MHz at 192mv/cm for 5 hrs; 2230MHz at 223mv/cm for 18 hrs; 2237MHz at 223mv/cm for 18 hrs; 2241MHz at 223mv/cm for 9 hrs; 2244MHz at 223mv/cm for 9 hrs. At the end of the culture period, the yeast cells were dried as described above.

To test the activated AS2.443 cells, a malodorous mixture was prepared as follow. 500 g of swine waste was collected and placed in a flask in which a partial vacuum is created. Five ml of hydrogen sulfide solution (70% concentrated) and 300 ml of water were added to and mixed with the animal waste under the partial vacuum. The partial vacuum is created by withdrawing about a volume of air which is about 20% of the volume of air above the surface of the extract in the flask. Nine such flasks were prepared. About 100 µl of activated AS2.443 yeast cells containing about 10⁹ cells were added to each of three flasks. To another three flasks were added the same number of non-activated AS2.443 yeast cells. No yeast cells were added to the remaining three flasks. The nine flasks were incubated for 37°C for 3 hours in an enclosed atmosphere with oxygen.

HPLC was used to analyze the amount of hydrogen sulfide in the mixture immediately after incubation. The amount of hydrogen sulfide was reduced by more than 82.3% relative to the control containing no yeasts. There were no significant changes in the samples containing non-activated yeasts and the blank control.

Accordingly to the invention, the odor caused by methylamine, dimethylamine, trimethylamine, ethylenediamine, and other aliphatic substituted amines can be reduced by yeasts cultured in the presence of an EM field that is in the range of 2180 to 2200 MHz. To demonstrate this aspect of the invention, cells of *Saccharomyces carlsbergensis* strain AS2.420 were cultured in a medium as described in Table 1 for 28 hours at 36°C. The culture started with 10⁹ cells per liter of medium. After the initial period of culture, the yeast cells were cultured in the presence of a series of eight EM fields in the order stated: 2182MHz at 176mv/cm for 51 hrs; 2188MHz at 176mv/cm for 51 hrs; 2191 MHz at 176mv/cm for 10 hrs; 2195MHz at 176mv/cm for 10 hrs; 2182MHz at 225mv/cm for 18 hrs; 2188MHz at 225mv/cm for 18 hrs; 2191MHz at 225mv/cm for 9 hrs; 2195MHz at 225mv/cm for 9 hrs. At the end of the culture period, the yeast cells were dried as described above.

To test the activated AS2.420 cells, a malodorous mixture was prepared as follow. 500 g of swine waste was collected and placed in a flask in which a partial vacuum is created. Three ml of ethylenediamine and dimethylamine each (80% concentrated) and 300 ml of water were added to and mixed with the animal waste under the partial vacuum. The partial vacuum is created by withdrawing about a volume of air which is about 20% of the volume of air above the surface of the extract in the flask. Nine such flasks were prepared. About 100 µl of activated AS2.420 yeast cells containing about 10⁹ cells were added to each of three flasks. To another three flasks were added the same number of non-activated AS2.420 yeast cells. No yeast cells were added to the remaining three flasks. The nine flasks were incubated for 37°C for 3 hours in an enclosed atmosphere with oxygen.

HPLC was used to analyze the amount of dimethylamine and ethylenediamine in the mixture immediately after incubation. The amount of dimethylamine and ethylenediamine was reduced by more than 87.4% relative to the control containing no yeasts. There were no significant changes in the samples containing non-activated yeasts and the blank control.

Accordingly to the invention, the odor caused by p-cresol and related compounds can be reduced by yeasts cultured in the presence of an EM field that is in the range of 2245 to 2265 MHz. To demonstrate this aspect of the invention, cells of Saccharomyces *carlsbergensis* strain AS2.4 were cultured in a medium as described in Table 1 for 28 hours at 36°C. The culture started with 10⁹ cells per liter of medium. After the initial period of culture, the yeast cells were cultured in the presence of a series of eight EM fields in the order stated: 2247MHz at 175mv/cm for 8 hrs; 2250MHz at 175mv/cm for 8 hrs; 2254MHz at 175mv/cm for 36 hrs; 2261MHz at 175mv/cm for 36 hrs; 2247MHz at 187mv/cm for 5 hrs; 2250MHz at 187mv/cm for 5 hrs; 2254MHz at 187mv/cm for 38 hrs; 2261MHz at 187mv/cm for 38 hrs. At the end of the culture period, the yeast cells were dried as described above.

To test the activated AS2.4 cells, a malodorous mixture was prepared as follow. 500 g of swine waste was collected and placed in a flask in which a partial vacuum is created. Four ml of concentrated p-cresol (95% pure) and 300 ml of water were added to and mixed with the animal waste under the partial vacuum. The partial vacuum is created by withdrawing about a volume of air which is about 20% of the volume of air above the surface of the extract in the flask. Nine such flasks were prepared. About 100 µl of activated AS2.4 yeast cells containing about 10⁹ cells were added to each of three flasks. To another three flasks were added the same number of non-activated AS2.4 yeast cells. No yeast cells were added to the remaining three flasks. The nine flasks were incubated for 37°C for 3 hours in an enclosed atmosphere with oxygen.

HPLC was used to analyze the amount of p-cresol in the mixture immediately after incubation. The amount of p-cresol was reduced by more than 78.9% relative to the control containing no yeasts. There were no significant changes in the samples containing non-activated yeasts and the blank control.

The above results show that each of the six yeast cell components can effectively reduce the concentration of malodorous compounds typically found in animal waste.

The present invention is not to be limited in scope by the specific embodiments described which are intended as single illustrations of individual aspects of the invention, and functionally equivalent methods and components are within the scope of the invention. Indeed various modifications of the invention, in addition to those shown and described herein will become apparent to those skilled in the art from the foregoing description and accompanying drawings. Such modifications are intended to fall within the scope of the appended claims.

## Claims

1. A biological composition comprising at least one of the following yeast cell components:
(a) a first yeast cell component comprising a plurality of yeast cells that are prepared by culturing the yeast cells in an electromagnetic field or a series of electromagnetic fields having a frequency in the range of 2442 to 2462 MHz and a field strength of 25 to 300 mV/cm;
(b) a second yeast cell component comprising a plurality of yeast cells that are prepared by culturing the yeast cells in an electromagnetic field or a series of electromagnetic fields having a frequency in the range of 2225 to 2245 MHz and a field strength of 25 to 300 mV/cm;
(c) a third yeast cell component comprising a plurality of yeast cells that are prepared by culturing the yeast cells in an electromagnetic field or a series of electromagnetic fields having a frequency in the range of 2320 to 2340 MHz and a field strength of 25 to 300 mV/cm;
(d) a fourth yeast cell component comprising a plurality of yeast cells that are prepared by culturing the yeast cells in an electromagnetic field or a series of electromagnetic fields having a frequency in the range of 2230 to 2250 MHz and a field strength of 25 to 300 mV/cm;
(e) a fifth yeast cell component comprising a plurality of yeast cells that are prepared by culturing the yeast cells in an electromagnetic field or a series of electromagnetic fields having a frequency in the range of 2180 to 2200 MHz and a field strength of 25 to 300 mV/cm; and
(f) a sixth yeast cell component comprising a plurality of yeast cells that are prepared by culturing the yeast cells in an electromagnetic field or a series of electromagnetic fields having a frequency in the range of 2245 to 2265 MHz and a field strength of 25 to 300 mV/cm.

2. The biological composition of claim 1 which comprises the yeast cell components of (a), (b), (c), (d), (e) and (f).

3. The biological composition of claim 1 or 2, wherein the yeast cells are cells of Saccharomyces.

4. The biological composition of claim 1 or 2, wherein the yeast cells are cells of *Saccharomyces cerevisiae* and *Saccharomyces carlsbergensis.*

5. The biological composition of any of claims 1 to 4, in which the yeast cells are dried.

6. An animal feed composition comprising the biological composition of any of claims 1 to 5, and pig feed.

7. An animal feed composition comprising the biological composition of any of claims 1 to 5, and chicken feed.

8. The animal feed composition of claim 6 or 7, in which 0.5% by weight is the biological composition of claim 1 or 2.

9. A method for preparing a biological composition, said method comprising culturing a plurality of yeast cells in an electromagnetic field or a series of electromagnetic fields having a frequency in the range of 2442 to 2462 MHz and a field strength of 25 to 300 mV/cm.

10. A method for preparing a biological composition, said method comprising culturing a plurality of yeast cells in an electromagnetic field or a series of electromagnetic fields having a frequency in the range of 2225 to 2245 MHz and a field strength of 25 to 300 mV/cm.

11. A method for preparing a biological composition, said method comprising culturing a plurality of yeast cells in an electromagnetic field or a series of electromagnetic fields having a frequency in the range of 2320 to 2340 MHz and a field strength of 25 to 300 mV/cm.

12. A method for preparing a biological composition, said method comprising culturing a plurality of yeast cells in an electromagnetic field or a series of electromagnetic fields having a frequency in the range of 2230 to 2250 MHz and a field strength of 25 to 300 mV/cm.

13. A method for preparing a biological composition, said method comprising culturing a plurality of yeast cells in an electromagnetic field or a series of electromagnetic fields having a frequency in the range of 2180 to 2200 MHz and a field strength of 25 to 300 mV/cm.

14. A method for preparing a biological composition, said method comprising culturing a plurality of yeast cells in an electromagnetic field or a series of electromagnetic fields having a frequency in the range of 2245 to 2265 MHz and a field strength of 25 to 300 mV/cm.

15. The method of any of claims 9 to 14, wherein said method further comprises culturing the plurality of yeast cells in one or more of the electromagnetic fields in a culture medium comprising porcine gastric juice, animal waste extract, wild hawthorn juice, and wild jujube juice.

16. A method of making an animal feed composition, said method comprising
(a) preparing one or more of the yeast cell components of claim 1,
(b) drying the yeast cell components of step (a) and
(c) mixing the dried yeast cells with animal feed.

17. The method of claim 16, wherein the drying step comprises (i) drying at a temperature not exceeding 65 °C for a period of time such that the yeast cells become dormant; and (b) drying at a temperature not exceeding 70°C for a period of time to reduce the moisture content to below 5%.

18. A method for reducing the odor of waste of an animal comprising feeding the animal for a period of time an animal feed composition comprising at least one of the following yeast cell components:
(a) a first yeast cell component comprising a plurality of yeast cells that are prepared by culturing the yeast cells in an electromagnetic field or a series of electromagnetic fields having a frequency in the range of 2442 to 2462 MHz and a field strength of 25 to 300 mV/cm;
(b) a second yeast cell component comprising a plurality of yeast cells that are prepared by culturing the yeast cells in an electromagnetic field or a series of electromagnetic fields having a frequency in the range of 2225 to 2245 MHz and a field strength of 25 to 300 mV/cm;
(c) a third yeast cell component comprising a plurality of yeast cells that are prepared by culturing the yeast cells in an electromagnetic field or a series of electromagnetic fields having a frequency in the range of 2320 to 2340 MHz and a field strength of 25 to 300 mV/cm;
(d) a fourth yeast cell component comprising a plurality of yeast cells that are prepared by culturing the yeast cells in an electromagnetic field or a series of electromagnetic fields having a frequency in the range of 2230 to 2250 MHz and a field strength of 25 to 300 mV/cm;
(e) a fifth yeast cell component comprising a plurality of yeast cells that are prepared by culturing the yeast cells in an electromagnetic field or a series of electromagnetic fields having a frequency in the range of 2180 to 2200 MHz and a field strength of 25 to 300 mV/cm; and
(f) a sixth yeast cell component comprising a plurality of yeast cells that are prepared by culturing the yeast cells in an electromagnetic field or a series of electromagnetic fields having a frequency in the range of 2245 to 2265 MHz and a field strength of 25 to 300 mV/cm.

19. The method of claim 18, wherein the animal feed composition comprises the yeast cell components of (a), (b), (c), (d), (e) and (f), and zeolite powder.

20. The method of claim 18 or 19 wherein said yeast cells are *Saccharomyces cerevisiae* cells.

21. The composition of claim 1 or 2, wherein the plurality of yeast cells used in preparing the first yeast cell component comprise cells of *Saccharomyces cerevisiae* AS2.558, wherein the plurality of yeast cells used in preparing the second yeast cell component comprise cells of *Saccharomyces cerevisiae* AS2.503, wherein the plurality of yeast cells used in preparing the third yeast cell component comprise cells of *Saccharomyces cerevisiae* AS2.504, wherein the plurality of yeast cells used in preparing the fourth yeast cell component comprise cells of *Saccharomyces carlsbergensis* AS2.443; wherein the plurality of yeast cells used in preparing the fifth yeast cell component comprise cells *of Saccharomyces cerevisiae* AS2.420; and wherein the plurality of yeast cells used in preparing the sixth yeast cell component comprise cells of *Saccharomyces carlsbergensis* AS2.4.

22. The animal feed composition of claim 6 or 7, wherein the plurality of yeast cells used in preparing the first yeast cell component comprise cells of *Saccharomyces cerevisiae* AS2.558, wherein the plurality of yeast cells used in preparing the second yeast cell component comprise cells of *Saccharomyces cerevisiae* AS2.503, wherein the plurality of yeast cells used in preparing the third yeast cell component comprise cells of *Saccharomyces cerevisiae* AS2.504; wherein the plurality of yeast cells used in preparing the fourth yeast cell component comprise cells of *Saccharomyces carlsbergensis* AS2.443; wherein the plurality of yeast cells used in preparing the fifth yeast cell component comprise cells of *Saccharomyces cerevisiae* AS2.420; and wherein the plurality of yeast cells used in preparing the sixth yeast cell component comprise cells of *Saccharomyces* carlsbergensis AS2.4.
